(19) Europäisches Patentamt
European Patent Office
Office européen des brevets

(11) EP 1 055 406 B1

(12) **FASCICULE DE BREVET EUROPEEN**

(45) Date de publication et mention de la délivrance du brevet:
**01.12.2004 Bulletin 2004/49**

(51) Int Cl.$^7$: **A61K 7/02**

(21) Numéro de dépôt: **00401118.5**

(22) Date de dépôt: **20.04.2000**

(54) **Composition sous forme d'émulsion huile-dans-eau et ses utilisations notamment cosmétiques**

Zusammensetzung vom Typ Öl-in-Wasser Emulsion und ihre Verwendung in der Kosmetik

Oil-in-water emulsion composition and use of said composition in cosmetics

(84) Etats contractants désignés:
**AT BE CH CY DE DK ES FI FR GB GR IE IT LI LU MC NL PT SE**

(30) Priorité: **26.05.1999 FR 9906659**

(43) Date de publication de la demande:
**29.11.2000 Bulletin 2000/48**

(73) Titulaire: **L'OREAL**
**75008 Paris (FR)**

(72) Inventeur: **Lorant, Raluca**
**94320 Thiais (FR)**

(74) Mandataire: **Rasson, Catherine**
**L'OREAL - D.I.P.I.**
**25-29 Quai Aulagnier**
**92600 Asnières (FR)**

(56) Documents cités:
**EP-A- 0 295 886**
**EP-A- 0 501 791**
**EP-A- 0 706 789**
**EP-A- 0 815 843**
**EP-A- 0 815 844**
**EP-A- 0 893 467**
**US-A- 5 599 533**

- **PATENT ABSTRACTS OF JAPAN vol. 1999, no. 04, 30 avril 1999 (1999-04-30) & JP 11 021227 A (KOSE CORP), 26 janvier 1999 (1999-01-26)**
- **PATENT ABSTRACTS OF JAPAN vol. 1999, no. 09, 30 juillet 1999 (1999-07-30) & JP 11 092335 A (KOSE CORP), 6 avril 1999 (1999-04-06)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).



Printed by Jouve, 75001 PARIS (FR)

## Description

La présente demande concerne une composition notamment cosmétique, sous forme d'émulsion huile-dans-eau contenant un organopolysiloxane solide élastomère réticulé comportant au moins un groupement oxyalkyléné et un polymère hydrosoluble ou gonflable dans l'eau. La demande se rapporte aussi à l'utilisation de ladite composition notamment dans le domaine cosmétique, et à l'utilisation de l'association de l'organopolysiloxane solide élastomère et du polymère hydrosoluble ou gonflable dans l'eau, pour la stabilisation d'une émulsion huile-dans-eau.

Pour diverses raisons liées en particulier à un meilleur confort d'utilisation (douceur, émollience et autres), les compositions cosmétiques actuelles se présentent le plus souvent sous la forme d'une émulsion du type huile-dans-eau (H/E) constituée d'une phase continue dispersante aqueuse et d'une phase discontinue dispersée huileuse ou d'une émulsion du type eau-dans-huile (E/H) constituée d'une phase continue dispersante huileuse et d'une phase discontinue dispersée aqueuse. Les émulsions H/E sont les plus demandées dans le domaine cosmétique du fait qu'elles comportent comme phase externe, une phase aqueuse, ce qui leur confère, lors de l'application sur la peau, un toucher plus frais, moins gras et plus léger que les émulsions E/H.

Les émulsions sont généralement stabilisées par des tensioactifs émulsionnants appropriés qui, grâce à leur structure amphiphile, se placent à l'interface huile/eau et stabilisent ainsi les gouttelettes dispersées. Ces émulsionnants présentent cependant l'inconvénient d'être pénétrants et potentiellement irritants pour la peau, les yeux et le cuir chevelu, notamment pour les sujets à peau sensible.

En outre, de telles émulsions peuvent avoir des propriétés cosmétiques et physico-chimiques insuffisantes (toucher huileux, instabilité dans le temps). Le fait d'augmenter le taux des tensioactifs ne résout pas généralement les problèmes mentionnés. La stabilité requise n'est pas toujours atteinte et les propriétés cosmétiques ne sont pas améliorées (toucher cireux, lourd, manque de fraîcheur à l'application). Par ailleurs, comme indiqué ci-dessus, il est aussi déconseillé d'utiliser un trop fort taux de tensioactif pour des raisons d'innocuité.

Une solution pour s'affranchir des phénomènes d'instabilité des émulsions H/E (crémage et déphasage) consiste à ajouter dans l'émulsion des agents épaississants dont la fonction est de créer, au sein de la phase aqueuse, une matrice gélifiée servant à figer les gouttelettes huileuses et assurant un maintien mécanique de l'ensemble de l'émulsion. Toutefois, cette solution présente l'inconvénient de ne pas permettre d'obtenir toutes les textures désirées et en particulier les textures légères, s'appliquant facilement et rapidement sur la peau sans laisser de film résiduel.

Par ailleurs, il a été envisagé de remplacer les tensioactifs par des polymères comportant dans leur chaîne une partie hydrophile et une partie hydrophobe constituée d'une chaîne grasse, tels que les copolymères d'alkyl-$C_{10}$-$C_{30}$-acrylate et d'acide acrylique ou méthacrylique, comme le produit "PEMULEN TR2" commercialisé par la société Goodrich. Cependant, ces polymères présentent l'inconvénient d'apporter un effet collant lors de l'application sur la peau et de ne pas permettre l'obtention d'une composition qui reste stable pendant une grande période de temps quand la quantité d'huile est trop importante.

Par ailleurs, le document EP-A-815844 décrit l'utilisation d'un polymère d'acide 2-acrylamido 2-méthylpropane sulfonique, réticulé et neutralisé, pour l'obtention d'émulsions exemptes de tensioactif. Toutefois, les émulsions obtenues ne sont pas parfaitement stables en présence d'un taux de phase huileuse important et par exemple supérieur à 12 %.

Le document JP-A-11-21227 décrit des émulsions E/H contenant un élastomère de silicone réticulé oxyéthyléné, un tensioactif et un polymère hydrosoluble.

Le document JP-A-11-92935 décrit des compositions solides sous forme d'émulsion E/H contenant un élastomère de silicone réticulé oxyéthyléné, et pouvant contenir des polymères hydrosolubles.

L'objectif de l'invention est de pouvoir réaliser des émulsions huile-dans-eau stables, c'est-à-dire qui ne se déphasent ni ne présentent de relargage d'huile, ne contenant éventuellement pas de tensioactif émulsionnant classiquement utilisé dans les émulsions H/E, et présentant de bonnes propriétés cosmétiques sans avoir les inconvénients de l'art antérieur, et ce quelle que soit la quantité d'huile contenue dans l'émulsion.

La demanderesse a découvert de façon inattendue que l'utilisation d'une association d'un organopolysiloxane solide élastomère réticulé et d'un polymère hydrosoluble ou gonflable dans l'eau, et notamment un polymère poly(acide 2-acrylamido 2-méthylpropane sulfonique) réticulé et neutralisé, permettait de réaliser des émulsions huile-dans-eau stables bien qu'éventuellement exemptes de tensioactif classiquement utilisé, cette stabilité persistant même quand le taux de phase huileuse est important. De plus, les compositions obtenues ont l'avantage de donner un effet de fraîcheur et de confort lors de l'application sur le peau, que l'on n'obtient pas avec les compositions de l'art antérieur.

La présente invention se rapporte à une composition sous forme d'émulsion huile-dans-eau comprenant une phase huileuse dispersée dans une phase aqueuse, caractérisée par le fait qu'elle contient dans un milieu physiologiquement acceptable, (1) au moins un organopolysiloxane solide élastomère réticulé comportant au moins un groupement oxyalkyléné et (2) au moins un polymère hydrosoluble ou gonflable dans l'eau.

L'émulsion obtenue est stable, même lorsqu'elle est exempte d'un tensioactif émulsionnant classiquement utilisé dans les émulsions H/E. L'invention a donc aussi pour objet l'utilisation de l'association d'un organopolysiloxane

solide élastomère réticulé et d'un polymère hydrosoluble ou gonflable dans l'eau, pour stabiliser une émulsion huile-dans-eau.

On entend par milieu physiologiquement acceptable dans la composition de l'invention, un milieu non toxique et susceptible d'être appliqué sur la peau (y compris l'intérieur des paupières), les lèvres, les ongles ou les cheveux d'êtres humains.

Par ailleurs, on entend par « solide élastomère » un matériau souple, déformable ayant des propriétés viscoélastiques et notamment la consistance d'une éponge ou d'une sphère souple. Son module d'élasticité est tel que ce matériau résiste à la déformation et possède une capacité limitée à l'extension et à la contraction. Ce matériau est capable de retrouver sa forme originelle suite à un étirement. Cet élastomère est formé de chaînes polymériques de haut poids moléculaire dont la mobilité est limitée par un réseau uniforme de points de réticulation.

Les organopolysiloxanes élastomères oxyalkylénés utilisés dans la composition de l'invention sont connus pour permettre la préparation d'émulsion E/H et il est tout à fait inattendue que l'on puisse les utiliser pour stabiliser une émulsion H/E contenant un taux élevé de phase huileuse, en association avec un polymère hydrosoluble ou gonflable dans l'eau.

Les organopolysiloxanes de la composition de l'invention contiennent un ou plusieurs groupements oxyalkylénés et en particulier oxyéthylénés (OE), par exemple de 1 à 40 motifs oxyalkylénés, de préférence 1 à 20 et mieux 10 à 20 motifs oxyalkylénés, pouvant former des chaînes polyoxyalkylènes et notamment polyoxyéthylénes. Ces groupements peuvent être pendants, en bout de chaîne ou destinés à lier deux parties de la structure siliconée. Les atomes de silicium portant ces groupements sont avantageusement au nombre d'environ 1 à 10 et mieux de 1 à 6.

Bien que l'invention concerne plus spécialement les organopolysiloxanes à groupement(s) oxyéthyléné(s) (à savoir les groupements ne comportant que des groupements oxyéthylénés comme groupements oxyalkylénés), elle peut aussi concerner les organopolysiloxanes à groupement(s) oxypropyléné(s), c'est-à-dire ne comportant que des groupements oxypropylénés comme groupements oxyalkylénés. Les organopolysiloxanes peuvent aussi comporter à la fois un ou plusieurs groupement(s) oxyéthyléné(s), 1 à 20 (OE) par exemple, et un ou plusieurs groupement(s) oxypropyléné(s) (OP), 0 à 20 par exemple ; ces organopolysiloxanes sont appelés aussi des organopolysiloxanes à groupement(s) alkyléthoxy-propyléné(s). De préférence, le nombre de groupements oxyéthylénés est supérieur au nombre de groupements oxypropylénés.

Par ailleurs, la structure siliconée formant le squelette polymérique de l'organopolysiloxane à groupement(s) oxyalkyléné(s) est avantageusement une structure polydiméthylsiloxane (PDMS) dont éventuellement une partie des groupes méthyle est substituée par des groupements alkyle en $C_2$ à $C_{30}$ et de préférence en $C_8$ à $C_{24}$, et mieux de $C_{10}$ à $C_{20}$ ou phényle, soit en bout de chaîne soit pendants.

Par ailleurs, l'organopolysiloxane à groupement(s) oxyalkyléné(s) peut comporter un ou plusieurs squelette (s) siliconé(s) relié(s) entre eux par un ou plusieurs groupements oxyalkylénés et de préférence oxyéthylénés tels que définis précédemment ou par un ou plusieurs groupements alkylénés, le nombre de groupement alkyléné allant de 1 à 20 et de préférence de 1 à 10. De préférence, il comporte au moins deux squelettes polymériques liés entre eux. Avantageusement, le ou les squelettes siliconés des organopolysiloxanes de la composition selon l'invention comportent de 26 à 80 atomes de silicium.

Les organopolysiloxanes élastomères de la composition de l'invention ne sont pas desséchants pour la peau et apportent de bonnes propriétés cosmétiques, notamment de douceur, de fraîcheur et de matité. Ces élastomères conduisent à des compositions confortables à l'application, de bon étalement, douces et non collantes au toucher. Ces propriétés cosmétiques sont dues, d'une part à la texture des organopolysiloxanes et, d'autre part à leurs propriétés comparables à celles de microéponges piégeant les milieux huileux et en particulier ceux de la composition et ceux sécrétés par la peau.

Les organopolysiloxanes élastomères utilisés dans la composition conforme à l'invention sont partiellement ou totalement réticulés et de structure tridimensionnelle. Inclus dans une phase huileuse, ils se transforment, selon le taux de phase huileuse utilisé, d'un produit d'aspect spongieux lorsqu'ils sont utilisés en présence de faibles teneurs en phase huileuse en un gel homogène en présence de quantités de phase huileuse plus élevées. La gélification de la phase huileuse par ces élastomères peut être totale ou partielle.

Les organopolysiloxanes élastomères utilisés selon l'invention peuvent se présenter sous forme de poudre ou de gel contenant un organopolysiloxane élastomère de structure tridimensionnelle, dispersé dans une phase huileuse. Cette phase huileuse, appelée encore phase grasse liquide, peut comprendre tout corps non aqueux ou mélange de corps non aqueux, liquide à température ambiante (environ 25°C) et à la pression atmosphérique (760 mm de Hg).

Les organopolysiloxanes élastomères utilisés selon l'invention peuvent être choisis parmi les polymères réticulés obtenus par réaction d'addition et de réticulation en milieu non aqueux, en présence d'un catalyseur notamment du type platine, d'au moins :

- (a) un premier organopolysiloxane (i) ayant au moins deux groupes vinyliques en position $\alpha$-$\omega$ de la chaîne siliconée ; et

- (b) un second organopolysiloxane (ii) ayant au moins un atome d'hydrogène lié à un atome de silicium par molécule et au moins un groupement oxyalkyléné notamment oxyéthyléné.

En particulier, l'organopolysiloxane (i) est choisi parmi les polydiméthylsiloxanes (PDMS) et est plus spécifiquement un $\alpha$-$\omega$-diméthylvinyl polydiméthylsiloxane. L'organopolysiloxane (ii) est choisi notamment parmi les polydiméthylsiloxanes comportant un ou plusieurs atome(s) d'hydrogène lié chacun à un atome de silicium, et un ou plusieurs groupements oxyéthylénés et éventuellement un ou plusieurs groupements oxypropylénés, liés à un atome de silicium via un radical alkylène ayant de 1 à 22 atomes de carbone.

Eventuellement les chaînes silicones des premiers et seconds organopolysiloxanes (i) et (ii) comportent des chaînes pendantes alkyle en $C_1$ à $C_6$ et/ou des chaînes aryle.

Les organopolysiloxanes élastomères de la composition selon l'invention se présentent avantageusement dans une phase huileuse avec laquelle il constitue un gel anhydre. Ce gel peut être notamment obtenu comme suit :

- (a) mélange du premier organopolysiloxane (i) et du second organopolysiloxane (ii) ;
- (b) ajout de la phase huileuse au mélange de l'étape (a) ; et
- (c) polymérisation du premier organopolysiloxane (i) et du second organopolysiloxane (ii) en phase huileuse en présence d'un catalyseur de platine.

La phase huileuse utilisée lors de la fabrication du gel anhydre contient une ou plusieurs huiles liquides à températures ambiante (25°C) choisies parmi les huiles hydrocarbonées et/ou les huiles de silicone. Avantageusement, la phase huileuse est une phase liquide siliconée, contenant une ou plusieurs huiles choisies parmi les polydiméthylsiloxanes (PDMS) à chaîne linéaire ou cyclique, liquides à température ambiante comportant éventuellement une chaîne alkyle ou aryle pendante ou en bout de chaîne, la chaîne alkyle ayant de 1 à 6 atomes de carbone.

Les organopolysiloxanes de l'invention sont en particulier obtenus selon le mode opératoire des exemples 3, 4 et 8 du document US-A-5,412,004 et des exemples du document US-A-5,811,487.

Selon un mode préféré de réalisation de l'invention, on utilise comme organopolysiloxanes celui commercialisé sous la référence KSG 21 par la société Shin Etsu ou le produit de l'exemple 3 (exemple de synthèse) du brevet US-A-5,412,004.

Le KSG 21 se présente sous forme d'un gel pâteux comprenant environ 28 % d'organopolysiloxane et 72 % d'huile de silicone (PDMS) ayant une viscosité de 6 cSt (soit $6.10^{-6}$ $m^2/s$).

Le produit de l'exemple 3 du brevet US-A-5,412,004 se présente sous la forme d'un gel pâteux contenant environ 33 % en poids d'organopolysiloxane réticulé à groupement(s) oxyéthyléné(s) et environ 67 % de PDMS 6 cSt (soit $6.10^{-6}$ $m^2/s$). L'organopolysiloxane contient environ 18 % d'oxyde d'éthylène en poids par rapport au poids total du polymère.

Le gel pâteux d'organosiloxane élastomère, utilisé dans la composition de l'invention, a un comportement rhéologique plastique présentant une viscosité sous faible cisaillement voisin de $10^{-3}$ $s^{-1}$ ou $10^{-4}$ $s^{-1}$, allant de $2.10^6$ Poises à $4.10^6$ Poises ($2.10^5$ Pa.s à $4.10^5$ Pa.s), et une viscosité dynamique de 15 à 50 Poises (1,5 à 5 Pa.s) pour une vitesse de cisaillement de 200 $s^{-1}$ à $t_{10\ minutes}$, mesurée avec un rhéomètre à contrainte imposée, RS 75 (Haake) à 25°C en géométrie cône/plan ; caractéristique du cône : 20 mm de diamètre, 1° d'angle et 40 µm de gap. Cet organopolysiloxane a, en outre, un comportement viscoélastique avec un caractère élastique dominant aux faibles valeurs de la contrainte de cisaillement, défini comme suit : 800 Pa < $G^*_{plateau}$ < 2 500 Pa avec $\delta_{plateau}$ voisin de 10°, $G^*_{plateau}$ représentant le module de rigidité (ou module complexe) c'est-à-dire la consistance, et étant mesuré à 1 Hz, et $\delta_{plateau}$ représentant l'élasticité (ou angle de perte). Il présente un point éclair d'environ 170°C à la pression atmosphérique.

Pour le produit de l'exemple 3 (exemple de synthèse) du document US-A-5,412,004, la viscosité dynamique, dans les conditions indiquées ci-dessus, est de 45 Poises (4,5 Pa.s).

De manière préférée, l'organopolysiloxane élastomère est introduit dans la phase huileuse de l'émulsion selon l'invention.

De façon préférentielle, le gel d'organopolysiloxane élastomère utilisé dans la composition de l'invention est présent dans la composition en une teneur allant d'environ 0,03 à 40 % et de préférence de 1,5 à 20 % en poids par rapport au poids total de la composition, ce qui correspond à un taux d'organopolysiloxane élastomère en matière active allant environ de 0,01 à 10 % en poids et mieux de 0,5 à 5 % en poids par rapport au poids total de la composition.

En particulier, les particules d'organopolysiloxane élastomère (en matière active) ont une taille allant de 0,1 à 500 µm, de préférence de 3 à 200 µm et mieux de 3 à 50 µm. Ces particules peuvent être sphériques, plates ou amorphes avec, de préférence, une forme sphérique.

Les polymères hydrosolubles ou gonflables dans l'eau, utilisés dans la composition de l'invention sont notamment des gélifiants et ils peuvent être choisis en particulier parmi les polymères carboxyvinyliques ; les copolymères acryliques ou méthacryliques ; les gommes naturelles ; les polysaccharides ; les polymères et copolymères d'acrylamide ; les copolymères de vinyl éther ; les polymères cationiques comme le polyquaternium. On peut aussi

utiliser un mélange de ces polymères.

Comme polymères carboxyvinyliques, on peut citer par exemple les polymères d'acide acrylique réticulés tels que les produits vendus sous les noms Carbopols 980, 981, 954, 2984 et 5984 (nom CTFA : carbomer) par la société GOODRICH ou les produits vendus sous les noms Synthalen M et Synthalen K par la société 3 VSA.

On peut citer aussi les homopolymères de diméthylaminoéthyl-méthacrylate quaternisé par le chlorure de méthyle, tels que les produits vendus sous les noms Salcare 95 et Salcare 96 par la société ALLIED COLLOIDS.

Comme copolymères acryliques ou méthacrylique, on peut citer notamment les copolymères d'acrylates d'alkyle en $C_{10}$-$C_{30}$ et d'acide acrylique ou méthacrylique ou de leur ester, vendus sous les dénominations Pemulen TR1, Pemulen TR2, Carbopol 1342 par la société GOODRICH (nom CTFA: Acrylates/C10-30 Alkyl Acrylate Crosspolymer).

On peut citer aussi les copolymères de diméthylamino-éthylméthacrylate quaternisé par le chlorure de méthyle et d'acrylamide tel que le produit Salcare SC92 vendu par ALLIED COLLOIDS ou le produit PAS 5194 vendu par HOECHST, et les copolymères réticulés de vinyl isodecanoate et d'acide acrylique et méthacrylique, tels que le produit Stabylen 30 vendu par la société 3M.

Comme gommes naturelles, on peut citer par exemple la gomme de xanthane, la gomme de gellane, la gomme de caroube.

Comme polysaccharides, on peut citer notamment les dérivés de cellulose, tels que par exemple l'hydroxypropylmethylcellulose, la carboxyméthylcellulose.

Comme copolymères d'acrylamide, on peut citer le copolymère réticulé d'acrylamide et d'acide 2-acrylamido 2-méthylpropane sulfonique, en particulier le mélange vendu sous le nom Sepigel 305 par la Société SEPPIC qui se présente sous forme d'une émulsion à environ 40 % de copolymère (nom CTFA : polyacrylamide/C13-14 isoparaffine/laureth-7).

Comme copolymères de vinyl éther, on peut citer par exemple les copolymères PVM/MA decadiène réticulés, tels que le Stabileze 06 vendu par la société ISP.

Comme polymères d'acrylamide, on peut citer notamment les polymères poly(acide 2-acrylamido 2-méthylpropane sulfonique) réticulés et pratiquement ou totalement neutralisés, caractérisés par le fait qu'ils comprennent, distribués de façon aléatoire :

a) de 90 à 99,9% en poids de motifs de formule générale (I) suivante :

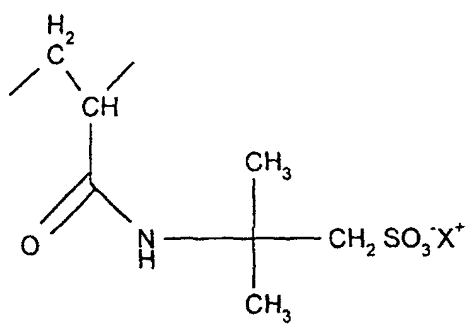

(I)

dans laquelle $X^+$ désigne un cation ou un mélange de cations, au plus 10% mol des cations $X^+$ pouvant être des protons $H^+$ ;

b) de 0,01 à 10% en poids de motifs réticulants provenant d'au moins un monomère ayant au moins deux doubles liaisons oléfiniques ; les proportions en poids étant définis par rapport au poids total du polymère.

De façon préférentielle, ces polymères poly(acide 2-acrylamido 2-méthylpropane sulfonique) comportent un nombre de motifs de formule (I) dans une quantité suffisamment élevée pour obtenir des particules de polymère dont le volume hydrodynamique en solution d'eau présente un rayon allant de 10 à 500 nm et dont la distribution est homogène et unimodale.

Dans la formule (I), $X^+$ représente un cation ou un mélange de cations choisis en particulier parmi un proton, un cation de métal alcalin, un cation équivalent de celui d'un métal alcalino-terreux ou l'ion ammonium.

Plus particulièrement, 90 à 100% mole des cations sont des cations $NH_4^+$ et 0 à 10% mole sont des protons ($H^+$).

Selon un mode préféré de réalisation de l'invention, on utilise comme polymères hydrosolubles ou gonflables dans l'eau, les polymères poly(acide 2-acrylamido 2-méthylpropane sulfonique) comprenant de 98 à 99,5 % en poids

de motifs de formule (I) et de 0,2 à 2 % en poids de motifs réticulants.

Les monomères de réticulation ayant au moins deux doubles liaisons oléfiniques sont choisis par exemple parmi le dipropylèneglycol-diallyléther, les polyglycol-diallyléthers, le triéthylèneglycol-divinyléther, l'hydroquinone-diallyl-éther, le tétrallyl-oxéthanoyle ou d'autres allyl ou vinyléthers alcools polyfonctionnels, le diacrylate de tétraéthylèneglycol, la triallylamine, le triméthylolpropane-diallyléther, le méthylene-bis-acrylamide ou le divinylbenzène.

Les monomères de réticulation ayant au moins deux doubles liaisons oléfiniques sont plus particulièrement choisis parmi ceux répondant à la formule générale (II) suivante :

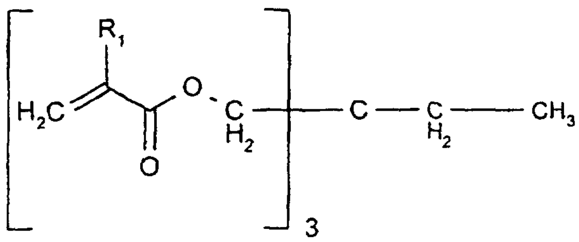

(II)

dans laquelle $R_1$ désigne un atome d'hydrogène ou un alkyle en $C_1$-$C_4$ et plus particulièrement méthyle. Le monomère de réticulation est de préférence le triméthylol propane triacrylate (composé de formule II où $R_1$ est l'hydrogène).

La réaction de polymérisation des polymères poly(acide 2-acrylamido 2-méthylpropane sulfonique) utilisés dans l'invention produit non seulement des chaînes linéaires mais aussi des molécules de polymère ramifiées ou réticulées. Ces molécules peuvent être caractérisées notamment par leur comportement rhéologique dans l'eau mais plus particulièrement par la diffusion de la lumière dynamique. Dans le cas de la caractérisation des molécules par la diffusion de la lumière dynamique, on mesure la distribution du volume hydrodynamique des structures du polymère. Les macromolécules dissoutes dans l'eau sont flexibles et entourées par une enveloppe de solvatation formée de molécules d'eau. Avec des polymères chargés comme ceux de l'invention, la taille des molécules dépend de la quantité de sel dans l'eau. Dans les solvants polaires, la charge uniforme le long de la chaîne principale du polymère conduit à une expansion importante de la chaîne polymérique. Le fait d'accroître la quantité de sel augmente la quantité d'électrolyte dans le solvant et écrante les charges uniformes du polymère. En plus des molécules transportées dans l'enveloppe de solvatation, les molécules de solvant sont fixées dans les cavités du polymère. Dans ce cas, les molécules de solvant font partie des macromolécules en solution et se déplacent à la même vitesse moyenne. Ainsi, le volume hydrodynamique décrit la dimension linéaire de la macromolécule et de ces molécules de solvatation.

Le volume hydrodynamique $v_h$ est déterminé par la formule suivante :

$$v_h = M/N_A \times (V_2 + dV_1)$$

avec :

- M désignant la masse en grammes de la macromolécule non-dissoute ;
- $N_A$ désignant le nombre d'Avogadro ;
- $V_1$ désignant le volume spécifique du solvant ;
- $V_2$ désignant le volume spécifique de la macromolécule ;
- d la masse en grammes du solvant qui est associé avec 1 gramme de macromolécule non-dissoute.

Si la particule hydrodynamique est sphérique, il est alors facile de calculer à partir du volume hydrodynamique le rayon hydrodynamique par la formule :

$$v_h = 4\Pi R^3/3$$

avec R désignant le rayon dynamique.

Les cas où les particules hydrodynamiques sont des sphères parfaites sont extrêmement rares. La majorité des polymères synthétiques impliquent des structures compactées ou des ellipsoïdes à haute excentricité. Dans ce cas, la détermination du rayon s'effectue sur une sphère qui est équivalente d'un point de vue frottement à la forme de la particule considérée.

En règle générale, on travaille sur des distributions de poids moléculaire et donc sur des distributions de rayon et de volume hydrodynamique. Pour les systèmes polydispersés, on doit calculer la distribution des coefficients de diffusion. De cette distribution, on en déduit les résultats relatifs à la distribution radiale et à la distribution des volumes hydrodynamiques.

Les volumes hydrodynamiques des polymères poly(acide 2-acrylamido 2-méthylpropane sulfonique) de l'invention sont en particulier déterminés par diffusion de la lumière dynamique à partir des coefficients de diffusion D selon STOKES-EINSTEIN de formule : $D = kT/6\Pi\eta R$ où k est la constante de Boltzmann, T la température absolue en degrés Kelvin, $\eta$ est la viscosité du solvant (eau) et R est le rayon hydrodynamique.

Ces coefficients de diffusion D sont mesurés selon la méthode de caractérisation d'un mélange de polymères par diffusion au LASER décrite dans les références suivantes :

(1) Pecora, R ; Dynamic Light Scattering ; Plenium Press, New York, 1976 ;
(2) Chu, B ; Dynamic Light Scattering ; Academic Press, New York, 1994 ;
(3) Schmitz, KS ; Introduction to Dynamic Light Scattering ; Academic Press, New York, 1990 ;
(4) Provincher S.W. ; Comp. Phys., 27, 213, 1982 ;
(5) Provincher S.W. ; Comp. Phys., 27, 229, 1982 ;
(6) ALV Laservertriebgesellschaft mbH, Robert Bosch Str. 47, D-63225 Langen, Germany ;
(7) ELS-Reinheimer Strasse 11, D-64846 Gross-Zimmern, Germany ;
(8) CHI WU et al ; Macromolecules, 1995, 28,4914-4919.

Les polymères poly(acide 2-acrylamido 2-méthylpropane sulfonique) particulièrement préférés sont ceux présentant une viscosité mesurée au viscosimètre BROOKFIELD, mobile 4, à une vitesse de rotation de 100 tours/minutes dans une solution d'eau à 2 % et à une température d'environ 25°C, supérieure ou égale à 1000 cps (ou 1000 mPa. s) et plus préférentiellement allant de 5000 à 40.000 cps (5000 à 40.000 mPa.s) et plus particulièrement de 6500 à 35.000 cps (6500 à 35.000 mPa.s).

Les poly(acide 2-acrylamido 2-méthylpropane sulfonique) réticulés utilisés dans la composition de l'invention peuvent être obtenus selon le procédé de préparation comprenant les étapes suivantes :

(a) on disperse ou on dissout le monomère acide 2-acrylamido 2-méthylpropane sulfonique sous forme libre dans une solution de tertio-butanol ou d'eau et de tertio-butanol ;

(b) on neutralise la solution ou la dispersion de monomère obtenue en (a) par une ou plusieurs bases minérales ou organiques, de préférence l'ammoniaque $NH_3$, dans une quantité permettant d'obtenir un taux de neutralisation des fonctions acides sulfoniques du polymère allant de 90 à 100% ;

(c) on ajoute à la solution ou dispersion obtenue en (b), le ou les monomères réticulants ;

(d) on effectue une polymérisation radicalaire classique en la présence d'amorceurs de radicaux libres à une température allant de 10 à 150°C ; le polymère précipitant dans la solution ou la dispersion à base de tertio-butanol.

Le poly(acide 2-acrylamido 2-méthylpropane sulfonique) réticulé utilisé dans la composition de l'invention peut être notamment le produit commercialisé par la société Hoechst sous la dénomination commerciale « Hostacerin AMPS » (nom CTFA : ammonium polyacryldimethyltauramide).

De manière préférée, le polymère hydrosoluble ou gonflable dans l'eau utilisé dans la composition de l'invention est introduit dans la phase aqueuse de l'émulsion selon l'invention.

Le polymère hydrosoluble ou gonflable dans l'eau utilisé dans la composition de l'invention est de préférence présent en une quantité en matière active allant de 0,1 à 10 % en poids, mieux de 0,2 à 5 % en poids et plus préférentiellement de 0,5 à 2 % en poids par rapport au poids total de la composition.

Outre les huiles éventuellement présentes dans le gel d'organopolysiloxane élastomère, la phase huileuse peut être de toute nature et comprendre des huiles, des cires ou des gommes solides à température ambiante, des corps gras pâteux, d'origine animale, végétale, minérale ou synthétique et leurs mélanges. Les huiles peuvent être volatiles à température ambiante et à la pression atmosphérique. Par huile volatile, on entend en particulier une huile susceptible de s'évaporer, en moins d'une heure, au contact de la peau ou des lèvres, ayant notamment une pression vapeur non nulle, en particulier allant de 0,133 Pa à 39996 Pa ($10^{-3}$ à 300 mm de Hg) (à température ambiante et pression atmosphérique) et de préférence supérieure à 39,996 Pa (0,3 mm de Hg).

Comme huiles utilisables dans la composition de l'invention, on peut citer notamment :

- les huiles hydrocarbonées d'origine animale, telles que le perhydrosqualène ;

- les huiles hydrocarbonées d'origine végétale, telles que les triglycérides liquides d'acides gras, par exemple les huiles de tournesol, de maïs, de soja, de courge, de pépins de raisin, de sésame, de noisette, d'abricot, de macadamia, de ricin, d'avocat, les triglycérides des acides caprylique/caprique comme ceux vendus par la société Stearineries Dubois ou ceux vendus sous les dénominations Miglyol 810, 812 et 818 par la société Dynamit Nobel ;
- les huiles de formule $R^1COOR^2$ dans laquelle $R^1$ représente le reste d'un acide gras supérieur comportant de 7 à 19 atomes de carbone et $R^2$ représente une chaîne hydrocarbonée ramifiée contenant de 3 à 20 atomes de carbone comme par exemple l'huile de Purcellin, le myristate d'isopropyle, des octanoates, décanoates ou ricinoléates d'alcools ou de polyalcools ;
- les hydrocarbures linéaires ou ramifiés, d'origine minérale ou synthétique, tels que les huiles de paraffine volatiles ou non et leurs dérivés, la vaseline, les polydécènes, le polyisobutène hydrogéné tel que le parléam ;
- les éthers de synthèse de formule $R^3OR^4$ dans laquelle $R^3$ est un radical alkyle en $C_3$ à $C_{19}$ et $R^4$ un radical alkyle en $C_3$ à $C_{20}$ ;
- des alcools gras comme l'octyldodécanol ou l'alcool oléique ;
- les huiles fluorées partiellement hydrocarbonées et/ou siliconées comme les perfluoropolyesters ;
- les huiles de silicone comme les polyméthylsiloxanes à chaîne siliconée linéaire ou cyclique, liquides ou pâteux à température ambiante, les phényldiméthicones, les phényltriméthicones, les polyméthylphénylsiloxanes, les alkylpolydiméthylsiloxanes avec une chaîne alkyle en $C_2$ à $C_{20}$ ;
- leurs mélanges.

La quantité de phase huileuse dans la composition de l'invention va de 5 à 40 % et mieux de 7 à 30 % en poids par rapport au poids total de la composition.

Avantageusement, l'émulsion de l'invention est exempte de tensioactif classiquement utilisé dans les H/E et elle présente de ce fait l'avantage de ne pas être irritante pour les peaux particulièrement sensibles. En outre, cette émulsion présente l'avantage de permettre l'incorporation d'actifs thermosensibles car elle peut être fabriquée à température ambiante.

De façon connue, les compositions de l'invention peuvent contenir des adjuvants habituels dans les domaines considérés, tels que des actifs hydrophiles ou lipophiles, des gélifiants et/ou épaississants classiques, des conservateurs, des antioxydants, des solvants, des charges, des matières colorantes, des agents basiques ou acides et encore des vésicules lipidiques. Ces adjuvants sont utilisés dans les proportions habituelles dans le domaine cosmétique, et par exemple de 0,01 à 30 % du poids total de l'émulsion, et ils sont, selon leur nature, introduits dans la phase aqueuse ou dans la phase huileuse de l'émulsion, ou encore dans des vésicules. Ces adjuvants ainsi que leurs concentrations doivent être tels qu'ils ne modifient pas la propriété recherchée pour l'émulsion de l'invention.

Comme solvants, on peut citer par exemple les mono-alcools linéaires ou ramifiés ayant de 1 à 8 atomes de carbone, comme l'éthanol, le propanol, le butanol, l'isopropanol, l'isobutanol ; les polyéthylène glycols ayant de 6 à 80 oxydes d'éthylène ; les polyols comme le propylène glycol, l'isoprène glycol et le butylène glycol.

Comme actifs, on peut citer par exemple les hydratants tels que les polyols comme la glycérine et le sorbitol ; les agents kératolytiques tels que les alpha-hydroxyacides ; l'acide salicylique et ses dérivés ; les vitamines comme la vitamine C, la vitamine A et la vitamine E et leurs dérivés et notamment leurs esters ; les dépigmentants ; les amincissants ; les filtres, et tout actif approprié pour le but final de la composition.

La composition de l'invention peut être notamment utilisée en application topique, en particulier dans les domaines cosmétique et dermatologique. Cette composition peut être plus ou moins fluide et avoir l'aspect d'une crème blanche ou colorée, d'une pommade, d'un lait, d'une lotion, d'un sérum, d'une pâte, d'une mousse. La composition de l'invention peut être appliquée par voie topique, sur le visage, y compris autour des yeux, sur le corps ainsi que sur le cuir chevelu des êtres humains.

La composition, objet de l'invention, trouve son application notamment dans un grand nombre de traitements cosmétiques de la peau, des lèvres et des cheveux, y compris le cuir chevelu, notamment pour le traitement, la protection, le soin, le démaquillage et/ou le nettoyage de la peau, des lèvres et/ou des cheveux, et/ou pour le maquillage de la peau et/ou des lèvres. Elle peut être destinée aussi au traitement des peaux sèches et/ou des lèvres sèches.

La composition selon l'invention peut, par exemple, être utilisée comme produit de soin, de démaquillage et/ou de nettoyage pour le visage sous forme de crème ou de lait, comme produit de maquillage (peau et lèvres) par incorporation de charges ou de colorants ou comme produits solaires par incorporation de filtres.

Aussi, l'invention a encore pour objet l'utilisation cosmétique de la composition telle que définie ci-dessus pour le traitement, la protection, le soin, le démaquillage et/ou le nettoyage de la peau, des lèvres et/ou des cheveux, et/ou pour le maquillage de la peau et/ou des lèvres.

L'invention a aussi pour objet un procédé de traitement cosmétique de la peau, y compris du cuir chevelu, des cheveux, et/ou des lèvres, caractérisé par le fait qu'on applique sur la peau, les cheveux et/ou les lèvres, une composition telle que définie ci-dessus.

L'invention a aussi pour objet l'utilisation de la composition telle que définie ci-dessus pour la fabrication d'une

composition destinée au soin des peaux sèches et/ou des lèvres sèches.

Les exemples qui suivent permettront de mieux comprendre l'invention, sans toutefois présenter un caractère limitatif. Les quantités indiquées sont en % en poids, sauf mention contraire.

**Exemple 1 : crème de soin**

*Phase aqueuse :*

- Hostacerin AMPS (vendu par la société Hoechst) 2 %
- Conservateurs 0,4 %
- Eau déminéralisée qsp 100 %

*Phase huileuse :*

- Isobutène hydrogéné 10 %
- Huile de silicone volatile 10 %
- KSG 21 (à 28 % de matière active) 3 %

(soit 0,84 % de matière active)

Mode opératoire : on prépare la phase aqueuse en dispersant sous agitation l'Hostacerin AMPS dans l'eau contenant les conservateurs et la glycérine. Puis, on disperse le KSG 21 dans la phase huileuse liquide et ensuite on émulsionne en dispersant sous agitation vigoureuse le mélange obtenu dans la phase aqueuse.

On obtient une crème stable, légèrement translucide très douce et fraîche sur la peau. Au microscope, la crème est fine et régulière.

**Exemple comparatif** :

On réalise une composition identique à celle de l'exemple 1 mais ne contenant pas de KSG21. On obtient une émulsion instable qui se révèle irrégulière et grossière au microscope.

**Exemple 2 : crème de soin**

*Phase aqueuse :*

- Sepigel 305 (vendu par la société Seppic) 2 %
- Conservateurs 0,4 %
- Glycérine 3 %
- Eau déminéralisée qsp 100 %

*Phase huileuse :*

- Huile de silicone volatile (cyclohexasiloxane) 7 %
- Huile d'abricot 5 %
- KSG 21 (à 28 % de matière active) 5 %

(soit 1,4 % de matière active)

Mode opératoire : on prépare la phase aqueuse en dispersant sous agitation le Sepigel 305 dans l'eau contenant les conservateurs et la glycérine. Puis, on disperse le KSG 21 dans la phase huileuse liquide et ensuite on émulsionne en dispersant sous agitation vigoureuse le mélange obtenu dans la phase aqueuse.

On obtient une crème légèrement translucide très douce et fraîche sur la peau.

**Exemple 3 : fond de teint matifiant**

*Phase aqueuse :*

- Hostacerin AMPS (vendu par la société Hoechst) 1,2 %
- Conservateurs 0,4 %
- Glycérine 3 %
- Eau déminéralisée qsp 100 %

*Phase huileuse :*

- Huile de silicone volatile (cyclohexasiloxane) 10 %
- KSG 21 (à 28 % de matière active) 3 %

(soit 0,84 % de matière active)

*Phase colorante :*

- Oxyde de titane 4 %
- Oxydes de fer 0,8 %

Mode opératoire : on prépare la phase aqueuse en dispersant sous agitation l'Hostacerin AMPS dans l'eau contenant les conservateurs et la glycérine. On disperse la phase colorante puis le KSG 21 dans la phase huileuse liquide, et ensuite on émulsionne en dispersant sous agitation vigoureuse le mélange obtenu dans la phase aqueuse.
On obtient une crème teintée non grasse et douce.

**Exemple 4 : soin solaire**

*Phase aqueuse* :

- Hostacerin AMPS (vendu par la société Hoechst) 2 %
- Conservateurs 0,4 %
- Glycérine 3 %
- Eau déminéralisée qsp 100 %

*Phase huileuse* :

- Huile de silicone volatile (cyclohexasiloxane) 5 %
- Octylmethoxycinnamate (filtre) 7 %
- Gel d'organosiloxane élastomère (exemple 3 du brevet US-5,412,004 à 33 % de matière active) 4 %

(soit 1,32 % de matière active)

Mode opératoire : on prépare la phase aqueuse en dispersant sous agitation l'Hostacerin AMPS dans l'eau contenant les conservateurs et la glycérine. On disperse le gel d'organosiloxane dans la phase huileuse liquide, et ensuite on émulsionne en dispersant sous agitation vigoureuse le mélange obtenu dans la phase aqueuse.
On obtient une crème douce et légère.

**Exemple 5 : Composition démaquillante pour peaux sensibles**

*Phase aqueuse :*

- Hostacerin AMPS (vendu par la société Hoechst) 1,5 %
- Conservateurs 0,4 %
- Glycérine 3 %
- Eau déminéralisée qsp 100 %

*Phase huileuse :*

- Huile de silicone volatile (cyclohexasiloxane) 5 %
- Palmitate d'octyle 5 %
- Gel d'organopolysiloxane (exemple 3 du brevet US-5,412,004) 2 %

(soit 0,66 % de matière active)

Mode opératoire : on prépare la phase aqueuse en dispersant sous agitation l'Hostacerin AMPS dans l'eau contenant les conservateurs et la glycérine. Puis, on disperse le gel d'organopolysiloxane dans la phase huileuse liquide et ensuite on émulsionne en dispersant sous agitation vigoureuse le mélange obtenu dans la phase aqueuse.

On obtient une émulsion riche qui démaquille en douceur les peaux délicates.

**Exemple 6 : Composition de soin**

*Phase aqueuse :*

- Sepigel 305 (vendu par la société Seppic) 2 %
- Conservateurs 0,4 %
- Glycérine 5 %
- Eau déminéralisée qsp 100 %

*Phase huileuse :*

- Huile de silicone volatile (cyclopentasiloxane) 10 %
- Isobutène hydrogéné 10 %
- Gel d'organopolysiloxane (exemple 3 du brevet US-5,412,004) 2 %

(soit 0,66 % de matière active)

Le procédé de préparation consiste à disperser sous agitation le Sepigel dans l'eau contenant les conservateurs et la glycérine. Puis, on disperse le gel d'organopolysiloxane dans la phase huileuse liquide et ensuite on émulsionne en dispersant sous agitation vigoureuse le mélange obtenu dans la phase aqueuse.

On obtient une crème douce, très agréable lors de l'application sur la peau qu'elle laisse souple et mate.

**Revendications**

1. Composition sous forme d'émulsion huile-dans-eau comprenant une phase huileuse dispersée dans une phase aqueuse, **caractérisée par le fait qu'**elle contient dans un milieu physiologiquement acceptable, (1) au moins un organopolysiloxane solide élastomère réticulé comportant au moins un groupement oxyalkyléné et (2) au moins un polymère hydrosoluble ou gonflable dans l'eau., et en que la quantité de phase huileuse va de 5 à 40 % en poids par rapport au poids total de la composition.

2. Composition selon la revendication 1, **caractérisée en ce qu'**elle est exempte de tensioactif.

3. Composition selon la revendication 1 ou 2, **caractérisée en ce que** l'organopolysiloxane élastomère comporte au moins un groupement oxyéthyléné.

4. Composition selon l'une quelconque des revendications précédentes, **caractérisée en ce que** l'organopolysiloxane élastomère ne comporte que des groupements oxyéthylénés comme groupements oxyalkylénés.

5. Composition selon l'une quelconque des revendications précédentes, **caractérisée en ce que** l'organopolysiloxane élastomère est obtenu par réaction d'addition et de réticulation en milieu non aqueux, en présence d'un catalyseur, d'au moins :

- un premier organopolysiloxane (i) ayant deux groupements vinyliques en position $\alpha$-$\omega$ de la chaîne siliconée par molécule ; et
- un second organopolysiloxane (ii) ayant au moins un atome d'hydrogène lié à un atome de silicium par molécule et au moins un groupement oxyalkyléné.

6. Composition selon la revendication précédente, **caractérisée en ce que** le premier organopolysiloxane (i) est choisi parmi les polydiméthylsiloxanes.

7. Composition selon la revendication précédente, **caractérisée en ce que** le premier organopolysiloxane (i) est un $\alpha$-$\omega$-diméthylvinyl polydiméthylsiloxane.

8. Composition selon l'une des revendications 5 à 7, **caractérisée en ce que** le second organopolysiloxane (ii) est choisi parmi les polydiméthylsiloxanes ayant un ou plusieurs atomes d'hydrogène et un ou plusieurs groupements oxyalkylénés liés à un atome de silicium via un radical alkylène ayant de 1 à 22 atomes de carbone.

9. Composition selon l'une quelconque des revendications 5 à 8, **caractérisée en ce que** l'organopolysiloxane est sous forme d'un gel obtenu selon les étapes suivantes :

- (a) mélange du premier et second organopolysiloxanes (i) et (ii) ;
- (b) ajout d'une phase huileuse au mélange de l'étape (a) ;
- (c) polymérisation du premier et second organopolysiloxanes (i) et (ii) en phase huileuse en présence d'un catalyseur de platine.

10. Composition selon la revendication précédente, **caractérisée en ce que** le gel a un module de rigidité $G^*_{plateau}$ mesuré à 1 Hz, défini comme suit : 800 Pa < $G^*_{plateau}$ < 2 500 Pa avec $\delta_{plateau}$ voisin de 10°, $\delta_{plateau}$ représentant l'élasticité.

11. Composition selon l'une des revendications précédentes, **caractérisée en ce que** l'organopolysiloxane élastomère représente de 0,01 à 10 % de matière active en poids par rapport au poids total de la composition.

12. Composition selon l'une quelconque des revendications précédentes, **caractérisée en ce que** le polymère hydrosoluble ou gonflable dans l'eau est choisi parmi les polymères carboxyvinyliques ; les copolymères acryliques ou méthacryliques ; les gommes naturelles ; les polysaccharides ; les polymères et copolymères d'acrylamide ; les copolymères de vinyl éther ; les polymères cationiques.

13. Composition selon l'une quelconque des revendications précédentes, **caractérisée en ce que** le polymère hydrosoluble ou gonflable dans l'eau est un poly(acide 2-acrylamido 2-méthylpropane sulfonique) réticulé et neutralisé à au moins 90% comprenant, distribués de façon aléatoire :

a) de 90 à 99,9% en poids de motifs de formule générale (I) suivante :

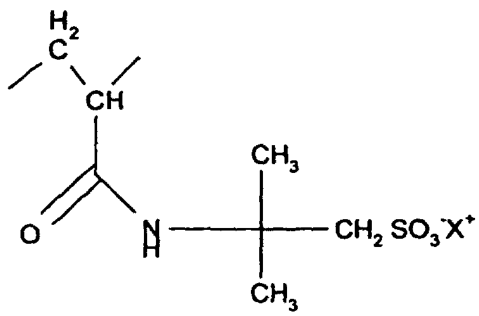

(I)

dans laquelle $X^+$ désigne un cation ou un mélange de cations, au plus 10% mol des cations $X^+$ pouvant être des protons $H^+$ ;

b) de 0,01 à 10% en poids de motifs réticulants provenant d'au moins un monomère ayant au moins deux doubles-liaisons oléfiniques ; les proportions en poids étant définis par rapport au poids total du polymère.

**14.** Composition selon la revendication 13, **caractérisée en ce que** le poly(acide 2-acrylamido 2-méthylpropane sulfonique) comporte de 98 à 99,5 % en poids de motifs de formule (I) et de 0,2 à 2 % en poids de motifs réticulants.

**15.** Composition selon la revendication 13 ou 14, **caractérisée en ce que** dans la formule (I) le cation $X^+$ est $NH_4^+$.

**16.** Composition selon l'une quelconque des revendications 13 à 15, **caractérisée en ce que** les monomères réticulants répondent à la formule générale (II) suivante :

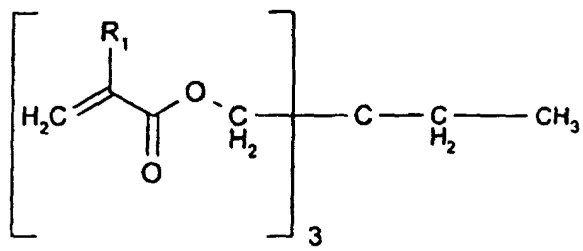

(II)

dans laquelle $R_1$ désigne hydrogène ou un alkyle en $C_1$-$C_4$.

**17.** Composition selon l'une quelconque des revendications 13 à 16, **caractérisée en ce que** le poly(acide 2-acrylamido 2-méthylpropane sulfonique) est réticulé par le triméthylol propane triacrylate.

**18.** Composition selon l'une quelconque des revendications précédentes, **caractérisée en ce que** la quantité de polymère hydrosoluble ou gonflable dans l'eau va de 0,1 à 10 % en poids de matière active par rapport au poids total de la composition.

**19.** Composition selon l'une des revendications précédentes, **caractérisée en ce que** la phase huileuse contient des huiles, des cires, des gommes, des corps gras pâteux et leurs mélanges.

**20.** Composition selon l'une quelconque des revendications précédentes, **caractérisée en ce que** la quantité de phase huileuse va de 7 à 30 % en poids par rapport au poids total de la composition.

**21.** Utilisation cosmétique de la composition selon l'une quelconque des revendications 1 à 20, pour le traitement, la protection, le soin, le démaquillage et/ou le nettoyage de la peau, des lèvres et/ou des cheveux, et/ou pour le maquillage de la peau et/ou des lèvres.

**22.** Procédé de traitement cosmétique de la peau, des cheveux, et/ou des lèvres, **caractérisé par le fait qu'**on applique sur la peau, les cheveux et/ou les lèvres, une composition selon l'une quelconque des revendications 1 à 20.

**23.** Utilisation de la composition selon l'une quelconque des revendications 1 à 20, pour la fabrication d'une composition destinée au soin des peaux sèches et/ou des lèvres sèches.

**24.** Utilisation de l'association d'un organopolysiloxane solide élastomère réticulé et d'un polymère hydrosoluble ou gonflable dans l'eau, pour stabiliser une émulsion huile-dans-eau.

## Patentansprüche

**1.** Zusammensetzung in Form einer Öl-in-Wasser-Emulsion, die eine Ölphase umfaßt, die in einer wäßrigen Phase dispergiert ist, **dadurch gekennzeichnet, dass** sie in einem physiologisch akzeptablen Medium (1) mindestens ein vernetztes elastomeres festes Polyorganosiloxan, das mindestens eine Oxyalkylengruppe enthält, und (2) mindestens ein wasserlösliches oder in Wasser quellfähiges Polymer enthält und dass die Menge der Ölphase im Bereich von 5 bis 40 Gew.-%, bezogen auf das Gesamtgewicht der Zusammensetzung, liegt.

**2.** Zusammensetzung nach Anspruch 1, **dadurch gekennzeichnet, dass** sie keinen grenzflächenaktiven Stoff enthält.

**3.** Zusammensetzung nach Anspruch 1 oder 2, **dadurch gekennzeichnet, dass** das elastomere Polyorganosiloxan mindestens eine Oxyethylengruppe enthält.

**4.** Zusammensetzung nach einem der vorhergehenden Ansprüche, **dadurch gekennzeichnet, dass** das elastomere Polyorganosiloxan ausschließlich Oxyethylengruppen als Oxyalkylengruppen enthält.

**5.** Zusammensetzung nach einem der vorhergehenden Ansprüche, **dadurch gekennzeichnet, dass** das elastomere Polyorganosiloxan durch die Additionsreaktion und Vernetzungsreaktion in nichtwässrigern Medium in Gegenwart eines Katalysators mindestens

- eines ersten Polyorganosiloxans (i), das zwei Vinylgruppen in $\alpha,\omega$-Position der Siliconkette pro Molekül aufweist; und
- eines zweiten Polyorganosiloxans (ii), das pro Molekül mindestens ein Wasserstoffatom, das mit einem Siliciumatom verbunden ist, und mindestens eine Oxyalkylengruppe aufweist,

erhalten wird.

**6.** Zusammensetzung nach dem vorhergehenden Anspruch, **dadurch gekennzeichnet, dass** das erste Polyorganosiloxan (i) unter den Polydimethylsiloxanen ausgewählt ist.

**7.** Zusammensetzung nach dem vorhergehenden Anspruch, **dadurch gekennzeichnet, dass** es sich bei dem ersten Polyorganosiloxan (i) um ein $\alpha,\omega$-Dimethylvinylpolydimethylsiloxan handelt.

**8.** Zusammensetzung nach einem der Ansprüche 5 bis 7, **dadurch gekennzeichnet, dass** das zweite Polyorganosiloxan (ii) unter den Polydimethylsiloxanen ausgewählt ist, die ein oder mehrere Wasserstoffatome und ein oder mehrere Oxyalkylengruppen aufweisen, die mit einem Siliciumatom über einen Alkylenrest verbunden sind, der 1 bis 22 Kohlenstoffatome aufweist.

**9.** Zusammensetzung nach einem der Ansprüche 5 bis 8, **dadurch gekennzeichnet, dass** das Polyorganosiloxan in Form eines Gels vorliegt, das durch die folgenden Verfahrensschritte erhalten wird:

- (a) Vermischen des ersten Polyorganosiloxans (i) und des zweiten Polyorganosiloxans (ii);
- (b) Zugeben einer Ölphase zu dem Gemisch aus Schritt (a);
- (c) Polymerisieren des ersten Polyorganosiloxans (i) und des zweiten Polyorganosiloxans (ii) in der Ölphase in Gegenwart eines Platin-Katalysators.

**10.** Zusammensetzung nach dem vorhergehenden Anspruch, **dadurch gekennzeichnet, dass** das Gel einen Steifigkeitsmodul $G^*_{Plateau,}$ der bei 1 Hz gemessen wird, aufweist, der wie folgt definiert ist: 800 Pa < $G^*_{Plateau}$ < 2500

Pa mit $\delta_{Plateau}$ in der Nähe von 10°, wobei $\delta_{Plateau}$ die Elastizität bedeutet.

**11.** Zusammensetzung nach einem der vorhergehenden Ansprüche, **dadurch gekennzeichnet, dass** das elastomere Polyorganosiloxan 0,01 bis 10 Gew.-% an wirksamer Substanz, bezogen auf das Gesamtgewicht der Zusammensetzung, ausmacht.

**12.** Zusammensetzung nach einem der vorhergehenden Ansprüche, **dadurch gekennzeichnet, dass** das wasserlösliche oder in Wasser quellfähige Polymer unter den Carboxyvinylpolymeren; den Acrylcopolymeren oder Methacrylcopolymeren; den natürlichen Gummen; den Polysacchariden; den Acrylamidpolymeren und Acrylamidcopolymeren; den Vinylethercopolymeren; den kationischen Polymeren ausgewählt ist.

**13.** Zusammensetzung nach einem der vorhergehenden Ansprüche, **dadurch gekennzeichnet, dass** das wasserlösliche oder in Wasser quellfähige Polymer eine vernetzte und zu mindestens 90 % neutralisierte Poly(2-acrylamido-2-methylpropansulfonsäure) ist, die zufällig verteilt enthält:

a) 90 bis 99,9 Gew.-% Einheiten der folgenden allgemeinen Formel (I)

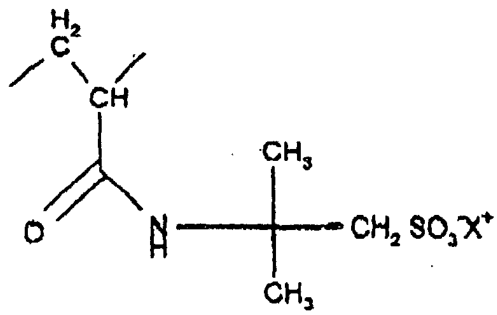

(I),

worin $X^+$ ein Kation oder ein Gemisch von Kationen bedeutet, wobei höchstens 10 Mol-% der Kationen $X^+$ Protonen $H^+$ sein können;

b) 0,01 bis 10 Gew.-% vernetzende Einheiten, die von mindestens einem Monomer stammen, das mindestens zwei olefinische Doppelbindungen aufweist;

wobei die Gewichtsanteile bezogen auf das Gesamtgewicht des Polymers definiert sind.

**14.** Zusammensetzung nach Anspruch 13, **dadurch gekennzeichnet, dass** die Poly(2-acrylamido-2-methylpropansulfonsäure) 98 bis 99,5 Gew.-% Einheiten der Formel (I) und 0,2 bis 2 Gew.-% vernetzende Einheiten enthält.

**15.** Zusammensetzung nach Anspruch 13 oder 14, **dadurch gekennzeichnet, dass** es sich bei dem Kation $X^+$ in der Formel (I) um $NH_4^+$ handelt.

**16.** Zusammensetzung nach einem der Ansprüche 13 bis 15, **dadurch gekennzeichnet, dass** die vernetzenden Monomere der folgenden allgemeinen Formel (II)

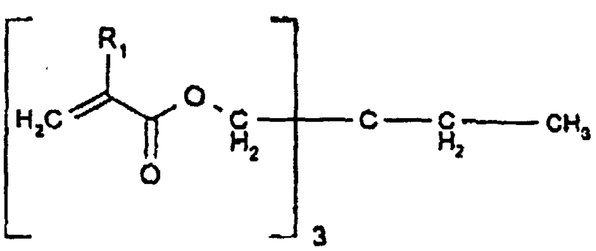

(II)

entsprechen, in der $R_1$ Wasserstoff oder $C_{1-4}$-Alkyl bedeutet,

**17.** Zusammensetzung nach einem der Ansprüche 13 bis 16, **dadurch gekennzeichnet, dass** die Poly(2-acrylamido-2-methylpropansulfonsäure) mit Trimethylolpropantriacrylat vernetzt ist.

**18.** Zusammensetzung nach einem der vorhergehenden Ansprüche, **dadurch gekennzeichnet, dass** die Menge des wasserlöslichen oder in Wasser quellfähigen Polymers im Bereich von 0,1 bis 10 Gew.-% an wirksamer Substanz, bezogen auf das Gesamtgewicht der Zusammensetzung, liegt.

**19.** Zusammensetzung nach einem der vorhergehenden Ansprüche, **dadurch gekennzeichnet, dass** die Ölphase Öle, Wachse; Gummen, pastöse Fettsubstanzen, Gemische dieser Materialien enthält.

**20.** Zusammensetzung nach einem der vorhergehenden Ansprüche, **dadurch gekennzeichnet, dass** die Menge der Ölphase im Bereich von 7 bis 30 Gew.-%, bezogen auf das Gesamtgewicht der Zusammensetzung, liegt.

**21.** Kosmetische Verwendung der Zusammensetzung nach einem der Ansprüche 1 bis 20 für die Behandlung, den Schutz, die Pflege, das Abschminken und/oder die Reinigung der Haut, der Lippen und/ oder der Haare und/ oder für das Schminken der Haut und/ oder der Lippen.

**22.** Verfahren zur kosmetischen Behandlung der Haut, der Haare und/ oder der Lippen, **dadurch gekennzeichnet, dass** eine Zusammensetzung nach einem der Ansprüche 1 bis 20 auf die Haut, die Haare und/oder die Lippen aufgetragen wird.

**23.** Verwendung der Zusammensetzung nach einem der Ansprüche 1 bis 20 für die Herstellung einer Zusammensetzung, die für die Pflege von trockener Haut und/oder trockenen Lippen vorgesehen ist.

**24.** Verwendung eines vernetzten elastomeren festen Polyorganosiloxans in Kombination mit einem wasserlöslichen oder in Wasser quellfähigen Polymer für die Stabilisierung einer Öl-in-Wasser-Emulsion.

## Claims

**1.** Composition in the form of an oil-in-water emulsion comprising an oily phase dispersed in an aqueous phase, **characterized in that** it comprises, in a physiologically acceptable medium, (1) at least one crosslinked solid organopolysiloxane elastomer comprising at least one oxyalkylene group and (2) at least one polymer which is soluble or swellable in water and **in that** the amount of oily phase ranges from 5 to 40% by weight with respect to the total weight of the composition.

**2.** Composition according to Claim 1, **characterized in that** it is devoid of surfactant.

**3.** Composition according to Claim 1 or 2, **characterized in that** the organopolysiloxane elastomer comprises at least one oxyethylene group.

**4.** Composition according to any one of the preceding claims, **characterized in that** the organopolysiloxane elastomer comprises only oxyethylene groups as oxyalkylene groups.

**5.** Composition according to any one of the preceding claims, **characterized in that** the organopolysiloxane elastomer is obtained by an addition and crosslinking reaction in a non-aqueous medium, in the presence of a catalyst, of at least:

- one first organopolysiloxane (i) having two vinyl groups in the $\alpha,\omega$-position of the silicone chain per molecule; and
- one second organopolysiloxane (ii) having at least one hydrogen atom bonded to a silicon atom per molecule and at least one oxyalkylene group.

**6.** Composition according to the preceding claim, **characterized in that** the first organopolysiloxane (i) is chosen from polydimethylsiloxanes.

**7.** Composition according to the preceding claim, **characterized in that** the first organopolysiloxane (i) is an $\alpha,\omega$)-dimethylvinylpolydimethylsiloxane.

**8.** Composition according to one of Claims 5 to 7, **characterized in that** the second organopolysiloxane (ii) is chosen from polydimethylsiloxanes having one or more hydrogen atoms and one or more oxyalkylene groups bonded to a silicon atom via an alkylene radical having from 1 to 22 carbon atoms.

**9.** Composition according to any one of Claims 5 to 8, **characterized in that** the organopolysiloxane is in the form of a gel obtained according to the following stages:

- (a) mixing the first and second organopolysiloxanes (i) and (ii);
- (b) adding an oily phase to the mixture of stage (a);
- (c) polymerizing the first and second organopolysiloxanes (i) and (ii) in the oily phase in the presence of a platinum catalyst.

**10.** Composition according to the preceding claim, **characterized in that** the gel has a rigidity modulus $G^*_{plate,}$ measured at 1 Hz, defined as follows: 800 Pa < $G^*_{plate}$ < 2500 Pa with $\delta_{plate}$ in the region of 10°, $\delta_{plate}$ representing the elasticity.

**11.** Composition according to one of the preceding claims, **characterized in that** the organopolysiloxane elastomer represents from 0.01 to 10% of active material by weight with respect to the total weight of the composition.

**12.** Composition according to any one of the preceding claims, **characterized in that** the polymer which is soluble or swellable in water is chosen from carboxyvinyl polymers; acrylic or methacrylic copolymers; natural gums; polysaccharides; acrylamide polymers and copolymers; vinyl ether copolymers; or cationic polymers.

**13.** Composition according to any one of the preceding claims, **characterized in that** the polymer which is soluble or swellable in water is a crosslinked poly(2-acrylamido-2-methylpropanesulphonic acid) neutralized to at least 90% comprising, distributed randomly:

a) from 90 to 99.9% by weight of units of following general formula (I):

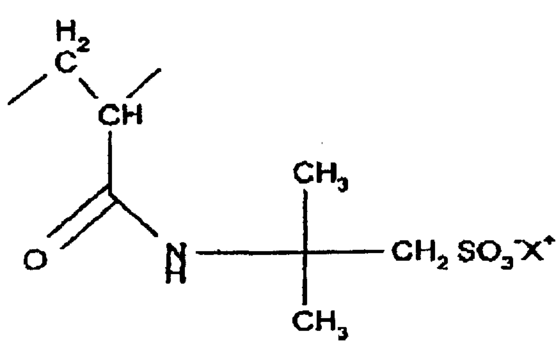

(I)

in which $X^+$ denotes a cation or a mixture of cations, it being possible for at most 10 mol% of the $X^+$ cations to be $H^+$ protons;

b) from 0.01 to 10% by weight of crosslinking units originating from at least one monomer having at least two olefinic double bonds; the proportions by weight being defined with respect to the total weight of the polymer.

**14.** Composition according to Claim. 13, **characterized in that** the poly(2-acrylamido-2-methylpropanesulphonic acid) comprises from 98 to 99.5% by weight of units of formula (I) and from 0.2 to 2% by weight of crosslinking units.

**15.** Composition according to Claim 13 or 14, **characterized in that**, in the formula (I), the $X^+$ cation is $NH_4^+$.

**16.** Composition according to any one of Claims 13 to 15, **characterized in that** the crosslinking monomers correspond to the following general formula (II):

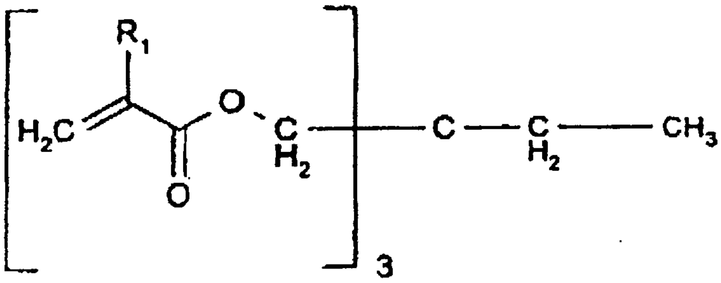

(II)

in which $R_1$ denotes hydrogen or a $C_1$-$C_4$ alkyl.

**17.** Composition according to any one of Claims 13 to 16, **characterized in that** the poly(2-acrylamido-2-methylpropanesulphonic acid) is crosslinked by trimethylolpropane triacrylate.

**18.** Composition according to any one of the preceding claims, **characterized in that** the amount of polymer which is soluble or swellable in water ranges from 0.1 to 10% by weight of active material with respect to the total weight of the composition.

**19.** Composition according to one of the preceding claims, **characterized in that** the oily phase comprises oils, waxes, gums, pasty fatty substances and their mixtures.

**20.** Composition according to any one of the preceding claims, **characterized in that** the amount of oily phase ranges from 7 to 30% by weight with respect to the total weight of the composition.

**21.** Cosmetic use of the composition according to any one of Claims 1 to 20 for treating, protecting, caring for, removing make-up from and/or cleansing the skin, lips and/or hair and/or for making up the skin and/or lips.

**22.** Process for the cosmetic treatment of the skin, of the hair and/or of the lips, **characterized in that** a composition according to any one of Claims 1 to 20 is applied to the skin, hair and/or lips.

**23.** Use of the composition according to any one of Claims 1 to 20 in the manufacture of a composition intended for caring for dry skin and/or dry lips.

**24.** Use of the combination of a crosslinked solid organopolysiloxane elastomer and of a polymer which is soluble or swellable in water for stabilizing an oil-in-water emulsion.